# DEMANDE DE BREVET EUROPEEN

(11) **EP 2 392 295 A1**
(43) Date de publication de la demande: **07.12.2011**
(21) Numéro de dépôt: 11290251.5
(22) Date de dépôt: 01.06.2011
(51) Int. Cl.: A61F 2/36

(54) **Implant prothétique à ancrage réversible**

(30) Priorité: 01.06.2010 FR 1002310
(71) Demandeur: Implant reduction, 91560 Crosne (FR)
(72) Inventeur: Herve, Jean-Louis, 91560 Crosne (FR); Perron, Claude-Henry, 91310 Leuville-sur-Orges (FR)
(74) Mandataire: Oudin, Stéphane

(57) **Abrégé**

La présente invention concerne un implant prothétique (1) pour la reconstruction osseuse comportant une tige (2) présentant une première extrémité (4) et une seconde extrémité (6), ladite tige (2) comporte un logement (8) longitudinal cylindrique débouchant sur la première (4) et seconde (6) extrémité de la tige (2), dans lequel un système d'ancrage (10) latéral et réversible de la tige (2) est intégré, remarquable en ce que ledit système d'ancrage (10) comporte une butée fixe (12), une butée mobile (14), au moins deux lumières (16) réalisées dans la paroi latérale de la tige (2) et débouchant sur le logement (8), une pièce d'ancrage (18) étant positionnée entre la butée fixe (12) et la butée mobile (14) au droit de chaque lumière (16), de manière telle que le rapprochement de la butée mobile (14) vers la butée fixe (12) entraine l'expansion radiale centrifuge des pièces d'ancrage (18) par rapport à l'axe longitudinal central du logement (8) et inversement.

Utilisation pour la réalisation notamment de prothèses fémorales et de clous chirurgicaux diaphysaire.

## Description

### Domaine technique :

La présente invention concerne un implant prothétique pour la reconstruction osseuse à ancrage réversible ainsi que son utilisation pour la réalisation d'implant fémoral de prothèse de hanche ou de clous chirurgicaux diaphysaires.

### Technique antérieure :

On rappellera en préambule que les os longs comportent globalement trois portions, celle de leur extrémité, dénommée épiphyse, dans laquelle le tissu osseux est spongieux, celle de la partie centrale ou médullaire, dénommée diaphyse, dans laquelle le tissu osseux est « dur » et la portion intermédiaire entre les deux précédentes, dénommée métaphyse.

Dans le domaine des implants fémoraux, on distingue les prothèses mise en place avec ou sans ciment. La présente invention fait partie de cette dernière catégorie. Pour ces implants destinés à être mis en place sans ciment, la partie de la tige de l'implant qui est insérée dans la portion médullaire de l'os du fémur est habituellement fixée à celui-ci au moyen de deux ou trois vis de fixation transversales qui traversent l'os et la tige. Ce mode de fixation s'avère parfois douloureux pour certains patients du fait de la pression exercée sur ces vis. Si les douleurs sont trop fortes, on procède alors à une ablation des vis, après validation de l'intervention suite à contrôle radiologique ou par IRM. Toutefois, cela génère une perte de stabilité de la prothèse qui présente alors un risque accru de migration. Ce risque existe également dans les cas de rupture des vis de fixation.

Des prothèses fémorales destinées à être fixées sans ciment et de manière réversible sont décrites dans plusieurs documents de l'art antérieur.

Ainsi, le brevet US 2685877 décrit une prothèse de tête de fémur dont la tige est destinée à être implantée dans l'épiphyse de l'os et ancrée dans celle-ci au moyen de deux ailettes montées en pivot dans le corps de la tige, qui pivotent vers l'extérieur de la tige sous l'actionnement d'une vis longitudinale en butée sur l'une des extrémités de ces ailettes. Un tel système, imaginé il y a plus de soixante ans, n'est pas du tout viable au plan chirurgical car la structure de l'os spongieux dans l'épiphyse ne permet pas d'obtenir un ancrage mécaniquement fiable et stable de la tige.

Par ailleurs, les documents FR 2653660 et EP 0385930 divulguent des prothèses dont la tige cylindrique est creuse et munie de fentes débouchantes sur l'extrémité distale de la tige, destinée à être implantée dans la diaphyse de l'os. Un noyau central d'expansion radial est monté sur un axe fileté à l'extrémité ouverte de la tige : son diamètre externe maximal est supérieur à celui de la tige, et comme il présente une forme à expansion progressive de type tronconique, son remontée dans le corps de la tige lors du vissage provoque la déformation radiale de la tige à son extrémité ouverte. Ces prothèses nécessitent l'aménagement dans l'os d'un tunnel de diamètre supérieur à celui du diamètre nominal au repos de la tige.

La présente invention a donc pour but de pallier ces inconvénients en proposant une alternative aux implants connus dont le mode d'ancrage sans ciment et dans la partie diaphysaire de l'os soit réversible, le moins traumatisant possible et sans risque de rupture.

### Résumé de l'invention

A cet égard, la présente invention concerne un implant prothétique pour la reconstruction osseuse comportant une tige présentant une première extrémité dite proximale et une seconde extrémité dite distale remarquable en ce que ladite tige comporte un logement longitudinal cylindrique débouchant sur la première et seconde extrémité de la tige, dans lequel un système d'ancrage latéral et réversible de la tige est intégré, ledit système d'ancrage comportant une butée fixe dite distale, une butée mobile dite proximale, au moins deux lumières réalisées dans la paroi latérale de la tige et débouchant sur le logement, une pièce d'ancrage étant positionnée entre la butée fixe et la butée mobile au droit de chaque lumière, de manière telle que le rapprochement de la butée mobile vers la butée fixe entraine l'expansion radiale centrifuge des pièces d'ancrage par rapport à l'axe longitudinal central du logement et inversement.

On comprend bien ainsi que le système d'ancrage selon l'invention ne nécessite avantageusement pas de percement transversal traumatisant des os. En outre, on saisi bien qu'en agissant sur la butée mobile, par exemple au moyen d'une clé introduite dans la tige de l'implant par l'extrémité proximale du logement, on va pouvoir facilement repositionner la tige.

Avantageusement, on exploitera la possibilité de jouer sur le degré de serrage de l'ancrage de la tige dans l'os pour générer des zones de redynamisation osseuse au niveau des moyens d'ancrage, du fait des micromouvements provenant du jeu micrométrique que l'on peut introduire.

### Brève description des figures

On décrira ci-après, à titre d'exemple non limitatif, une forme d'exécution préférentielle de l'implant prothétique selon l'invention, sous la forme d'un implant fémoral, du type sans ciment, en référence aux dessins annexés sur lesquels .
- la figure 1 est une vue avant en élévation d'un implant fémoral ;
- la figure 2 est une vue partielle en coupe de la tige de l'implant selon l'axe II-II' représenté à la figure 1 ;
- la figure 3 est une vue de détail de la figure 2 représentant le système d'ancrage déployé ;
- la figure 4 est une vue en coupe de la tige de l'implant selon l'axe IV-IV' représenté à la figure 1 ;
- la figure 5 est une vue de détail similaire à celle de la figure 3, le système d'ancrage étant rentré ;
- la figure 6 est une vue en élévation de la butée mobile du système d'ancrage ;
- la figure 7 est une vue en élévation de la butée fixe du système d'ancrage ;
- la figure 8 est une en élévation d'une pièce d'ancrage ;
- la figure 9 est une en coupe transversale de la pièce d'ancrage selon l'axe IX-IX' représenté à la figure 8.

### Description détaillée de l'invention

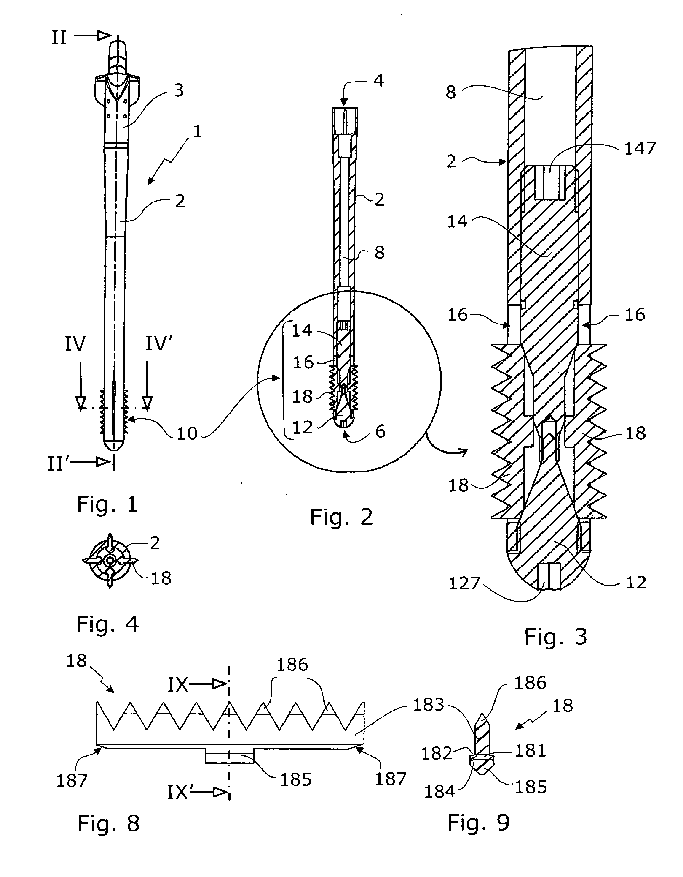

En référence à la figure 1, l'implant 1 fémoral comprend une tige 2 et une tête 3 solidaires et habituellement reliées par un col à emmanchement conique. La tige 2 présente une première extrémité 4 dite proximale et une seconde extrémité 6 dite distale.

Vers son extrémité distale, la tige 2 présente une forme cylindrique.

Selon une caractéristique essentielle de l'invention, ladite tige 2 comporte un logement 8 longitudinal cylindrique débouchant sur la première 4 et seconde 6 extrémité de la tige 2, dans lequel un système d'ancrage 10 latéral et réversible de la tige 2 est intégré.

Le logement 8 peut déboucher directement aux deux extrémités 4,6 de la tige 2 ou bien, comme illustré, il peut être prolongé par une succession de plusieurs logements cylindriques alignés et coaxiaux, de diamètres différents, afin d'optimiser la résistance mécanique de la tige 2 ainsi creusée tout en permettant l'introduction d'une clé de serrage par les extrémités pour l'actionnement du système d'ancrage 10.

Selon un aspect important de l'invention, la tige 2 est indéformable ; plus précisément, elle n'est pas déformée par l'actionnement du système d'ancrage 10 : ce dernier ne fait pas varier le diamètre externe de la tige de l'implant.

En référence aux figures 2 à 5, le système d'ancrage 10 comporte une butée fixe 12 dite distale, une butée mobile 14 dite proximale, au moins deux lumières 16 réalisées dans la paroi latérale de la tige 2 et débouchant sur le logement 8, une pièce d'ancrage 18 étant positionnée entre la butée fixe 12 et la butée mobile 14, au droit de chaque lumière 16, de manière telle que le rapprochement de la butée mobile 14 vers la butée fixe 12 entraine l'expansion radiale centrifuge des pièces d'ancrage 18 par rapport à l'axe longitudinal central du logement 8 et inversement.

Selon une variante préférée, les lumières 16 sont distribuées régulièrement à la circonférence du logement par rapport à son axe longitudinal central. Ces lumières sont au moins au nombre de deux. De préférence, elles sont au nombre de trois ; plus préférentiellement, au nombre de quatre. Bien sûr, l'Homme de l'Art pourra mettre en oeuvre cinq ou six, ou plus, lumières 16, en fonction des dimensions et contraintes applicables, sans sortir du cadre de la présente invention.

Les lumières 16 ne sont pas ouvertes sur l'une ou l'autre des extrémités proximale 4 ou distale 6 de la tige 2. Elles peuvent revêtir n'importe quelle forme. Par exemple, comme illustré, elles peuvent présenter une forme de rectangle. Les lumières 16 sont positionnées entre la butée fixe 12 et la butée mobile 14, lorsque cette dernière est en butée du coté de la première extrémité 4 proximale de la tige 2.

En référence aux figures 8 et 9, chaque pièce d'ancrage 18 comporte une embase 181 parallélépipédique, munie sur sa face externe 182 d'une lame 183 issue perpendiculairement à sa surface et comportant des moyens d'ancrage, tandis que la face interne 184 opposée comporte une butée 185 optionnelle de positionnement. Avantageusement, la lame 183 est positionnée de façon médiane sur la face externe 181 de sorte à procurer de part et d'autre un épaulement qui servira de butée de course de la pièce d'ancrage 18 lors du déploiement du système d'ancrage 10. Selon une variante non représentée, les pièces d'ancrage 18 sont dépourvues de butée 185 de positionnement.

On comprend bien que le dimensionnement des pièces d'ancrage 18 sera corrélé à celui des lumières 16 de sorte que la lame 183 puisse passer par lesdites lumières et être en saillie de la surface externe de la tige 2 lorsque le système d'ancrage 10 est déployé. En outre, afin d'assurer un ancrage fort au plan mécanique, on choisira de conférer une certaine longueur aux pièces d'ancrage 18.

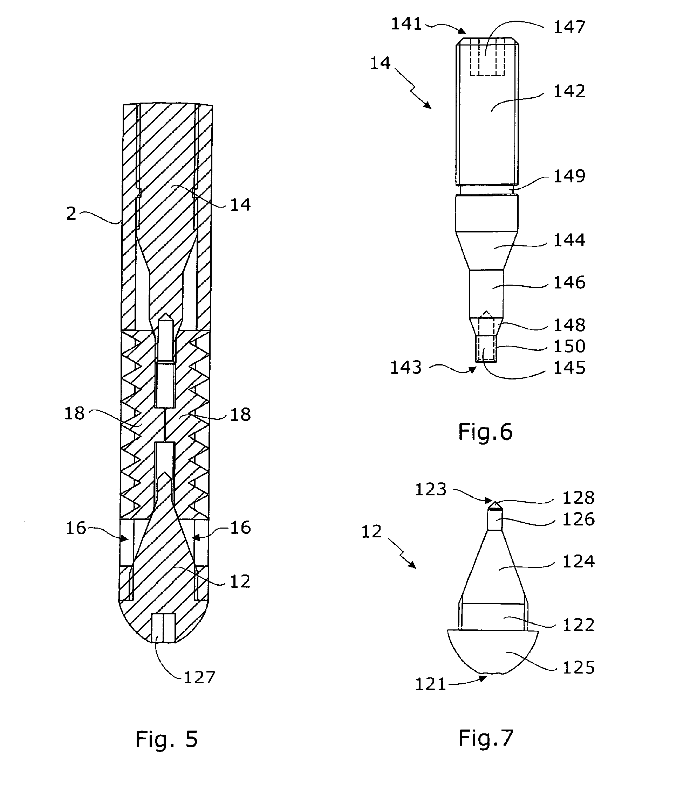

Les butées optionnelles de positionnement 185 de chacune des pièces d'ancrage 18 sont en contact les unes des autres lorsque le système d'ancrage est en position neutre, comme illustré à la figure 5. En outre, elles servent avantageusement de point d'appui supplémentaire lors du déploiement du système d'ancrage 10. Cette caractéristique sera détaillée plus loin.

Selon une variante préférée, les moyens d'ancrage sont des dents 186 ; l'Homme de l'Art pourra donner aux dents toute forme permettant d'assurer une prise optimale. En outre, on pourra envisager pour les moyens d'ancrage tout autre moyen connu assurant cette fonction d'ancrage, comme une surface munie de stries par exemple.

Selon une autre caractéristique, les bords 187 dans le sens de la largeur de la face interne 184 de l'embase 181 des pièces d'ancrage 18 sont biseautés. On reviendra plus loin sur cette caractéristique.

Selon une caractéristique importante de l'invention, le logement 8 est taraudé sur au moins une partie de sa longueur. Cela permet le positionnement des butées fixe 12 et mobile 14 dans le logement 8.

Selon une caractéristique avantageuse de l'invention, la butée mobile 14 présente au moins une section conique ou tronconique.

En référence à la figure 6, on décrira maintenant une variante d'exécution préférée de la butée mobile 14.

La butée mobile 14 comporte depuis son extrémité proximale 141 vers son extrémité distale 143 une première section 142 cylindrique, munie d'un filetage sur une partie au moins de sa longueur, dont le diamètre correspond à celui du diamètre du logement 8, une seconde section 144 tronconique, elle-même contigüe à une troisième section 146 cylindrique, suivie d'une quatrième section 148 tronconique qui est terminée à l'extrémité distale 143 de la butée mobile 14 par une cinquième section 150 cylindrique.

En outre, un logement 145 cylindro-conique débouchant à l'extrémité 143 est aménagé dans la quatrième et la cinquième section. Ce logement 145 est destiné à accueillir l'extrémité proximale de la butée fixe 12 lorsque le système d'ancrage est déployé et que la butée mobile a été rapprochée de la butée fixe.

Une empreinte creuse 147 est aménagée dans la première section 142 afin de permettre le vissage de la butée mobile 12 dans le logement 8. L'empreinte 147 pourra par exemple être hexagonale.

Une rainure annulaire 149 est aménagée dans la première section cylindrique 142 de la butée mobile 14, délimitant la limite distale de la partie filetée. Cette rainure annulaire 149 permet le dégagement du filet lors de l'usinage de la pièce : on optimise ainsi le positionnement de la butée mobile 14 dans le logement 8.

Selon une caractéristique avantageuse de l'invention, la butée fixe 12 présente au moins une section conique ou tronconique.

En référence à la figure 7, on décrira une variante préférée de réalisation de la butée fixe 12.

La butée fixe 12 comporte depuis son extrémité distale 121 vers son extrémité proximale 123 une première section 122 cylindrique, munie d'un filetage, dont le diamètre correspond à celui du diamètre du logement 8, contigüe à une seconde section 124 tronconique, elle-même contigüe à une troisième section 126 cylindrique qui est terminée à l'extrémité proximale 123 de la butée fixe 12 par une quatrième section 128 conique.

Dans la configuration de réalisation représentée, le système d'ancrage 10 est positionné à proximité de l'extrémité distale 6 de la tige 2. La butée fixe 12 comporte en outre à son extrémité distale 121 un chapeau hémisphérique 125, chapeautant la première section cylindrique 122, dont le diamètre correspond au diamètre externe de la tige 2, de sorte à former un bouchon à la seconde extrémité 6 de cette dernière.

En outre, une empreinte creuse 127 est aménagée dans la partie hémisphérique 125 afin de permettre le vissage de la butée fixe 12 dans le logement 8. L'empreinte 127 pourra par exemple être hexagonale.

On comprend bien que dans d'autres configurations de réalisation de l'implant prothétique selon l'invention, dans lesquelles le système d'ancrage ne serait plus situé à la proximité immédiate de l'extrémité distale 6 de la tige 2, la butée fixe 12 ne comporterait alors pas de chapeau hémisphérique 125, l'empreinte creuse 127 serait aménagée dans la première section 122 cylindrique et l'implant 1 serait muni d'un bouchon additionnel afin d'obturer le logement 8 à l'extrémité distale 6 de la tige 2.

En référence aux figures 5 et 3, on expliquera le déploiement du système d'ancrage 10. En position neutre, tel que représenté à la figure 5, les pièces d'ancrage 18 sont en appui par leur bords 187 biseautés sur la seconde section tronconique de la butée mobile et sur la seconde section tronconique de la butée fixe. En outre, lorsqu'elles en sont munies, les pièces d'ancrage 18 sont en contact entre elles au niveau des butées 185. Lorsque l'on procède au rapprochement de la butée mobile 14 de la butée fixe 12 par vissage de celle-ci, les pièces d'ancrage 18 vont glisser progressivement sur les surfaces de contact tronconique des butées fixe 12 et mobile 14 et rester globalement parallèles à la surface interne du logement 8. En fin de course, les butées 185 sont en appui sur la troisième section cylindrique de la butée mobile. On obtient ainsi trois points d'appui répartis sur la longueur des pièces d'ancrage 18, ce qui évite avantageusement leur déformation par flexion lorsqu'elles sont déployées et ancrées dans l'os, d'autant plus si la longueur des pièces d'ancrage 18 est importante. La mise en oeuvre de pièces d'ancrage 18 dépourvues de butée 185 conduit à un appui sur deux points, qui peut suffire à assurer un ancrage dans l'os de l'implant, n'est toutefois pas optimal au plan mécanique. On préférera de ce fait recourir à la variante des pièces d'ancrage munies desdites butées 185.

Enfin, toujours en fin de course, la quatrième section 128 conique et la troisième section 126 cylindrique de la butée fixe 12 sont engagées dans le logement 145 de la butée mobile 14.

### Application industrielle

L'implant prothétique selon l'invention trouvera une application particulièrement préférée dans la réalisation de prothèses fémorales ou de clous chirurgicaux diaphysaires. En particulier, les prothèses fémorales ainsi obtenues, à implantation sans ciment, pourront être utilisées en tant que prothèse de reprise mais aussi de première intention ou de remplacement de prothèse de reprise défectueuse, par exemple en cas de rupture des vis de fixation transversale.

Enfin, il va de soi que la présente invention n'est pas limitée à l'exemple de réalisation préférentiel et à la mise en oeuvre décrits, et elle peut être modifiée ou adaptée en fonction des besoins ou des exigences particulières, sans pour autant sortir du cadre de l'invention.

## Revendications

1. Implant prothétique (1) pour la reconstruction osseuse comportant une tige (2) présentant une première extrémité (4) dite proximale et une seconde extrémité (6) dite distale, ladite tige (2) comporte un logement (8) longitudinal cylindrique débouchant sur la première (4) et seconde (6) extrémité de la tige (2), dans lequel un système d'ancrage (10) latéral et réversible de la tige (2) est intégré, **caractérisé en ce que** ledit système d'ancrage (10) comporte une butée fixe (12) dite distale, une butée mobile (14) dite proximale, au moins deux lumières (16) réalisées dans la paroi latérale de la tige (2) et débouchant sur le logement (8), une pièce d'ancrage (18) étant positionnée entre la butée fixe (12) et la butée mobile (14) au droit de chaque lumière (16), de manière telle que le rapprochement de la butée mobile (14) vers la butée fixe (12) entraine l'expansion radiale centrifuge des pièces d'ancrage (18) par rapport à l'axe longitudinal central du logement (8) et inversement.

2. Implant prothétique (1) selon la revendication 1 **caractérisé en ce que** les lumières (16) ne débouchent pas sur la première extrémité (4) ou la seconde extrémité (6) de la tige (2).

3. Implant prothétique (1) selon l'une quelconque des revendications 1 ou 2 **caractérisé en ce que** la pièce d'ancrage (18) comporte une embase (181) parallélépipédique, munie sur sa face externe (182) d'une lame (183) issue perpendiculairement à sa surface et comportant des moyens d'ancrage

4. Implant prothétique (1) selon la revendication 3 **caractérisé en ce que** la face interne (184) opposée à la face externe (182) de la pièce d'ancrage (18) comporte une butée (185) de positionnement.

5. Implant prothétique (1) selon l'une quelconque des revendications 3 ou 4 **caractérisé en ce que** les moyens d'ancrage sont des dents (186).

6. Implant prothétique (1) selon l'une quelconque des revendications 3 à 5 **caractérisé en ce que** les bords (187) dans le sens de la largeur de la face interne (184) de l'embase (181) sont biseautés.

7. Implant prothétique (1) selon l'une quelconque des revendications 1 à 6 **caractérisé en ce que** le logement (8) est taraudé sur au moins une partie de sa longueur.

8. Implant prothétique (1) selon l'une quelconque des revendications 1 à 7 **caractérisé en ce que** la butée mobile (14) présente au moins une portion conique ou tronconique.

9. Implant prothétique (1) selon l'une quelconque des revendications 1 à 8 **caractérisé en ce que** la butée fixe (12) présente au moins une portion conique ou tronconique.

10. Implant prothétique (1) selon l'une quelconque des revendications précédentes **caractérisé en ce que** la butée fixe (12) comporte depuis son extrémité distale (121) vers son extrémité proximale (123) une première section (122) cylindrique, munie d'un filetage sur une partie au moins de sa longueur, dont le diamètre correspond à celui du diamètre du logement (8), contigüe à une seconde section (124) tronconique, elle-même contigüe à une troisième section (126) cylindrique qui est terminée à l'extrémité proximale (123) de la butée fixe (12) par une quatrième section (128) conique.

11. Implant prothétique (1) selon l'une quelconque des revendications précédentes **caractérisée en ce que** la butée mobile (14) comporte depuis son extrémité proximale (141) vers son extrémité distale (143) une première section (142) cylindrique, munie d'un filetage, dont le diamètre correspond à celui du diamètre du logement (8), contigüe à une seconde section (144) tronconique, elle-même contigüe à une troisième section (146) cylindrique, suivie d'une quatrième section (148) tronconique qui est terminée à l'extrémité proximale (143) de la butée mobile (14) par une cinquième section (150) cylindrique.

12. Utilisation d'un implant prothétique (1) selon l'une quelconque des revendications 1 à 11 pour réaliser une prothèse fémorale.

13. Utilisation d'un implant prothétique (1) selon l'une quelconque des revendications 1 à 11 pour réaliser des clous chirurgicaux diaphysaires.
